# FASCICULE DE BREVET EUROPEEN

(11) **EP 2 575 914 B1**
(45) Date de publication et mention de la délivrance du brevet: **11.06.2014**
(21) Numéro de dépôt: 11727550.3
(22) Date de dépôt: 31.05.2011
(51) Int. Cl.: A61L 27/54, A61F 2/02, B01D 67/00

(54) **MEMBRANE FONCTIONNALISÉE POUR CHAMBRE D'ENCAPSULATION DE CELLULES PRODUISANT AU MOINS UNE SUBSTANCE D'INTÉRÊT THÉRAPEUTIQUE ET ORGANE BIOARTIFICIEL COMPRENANT UNE TELLE MEMBRANE.**
FUNKTIONALISIERTE MEMBRAN FÜR EINE KAMMER ZUR VERKAPSELUNG VON ZELLEN ZUR HERSTELLUNG VON MINDESTENS EINEM STOFF VON THERAPEUTISCHEM INTERESSE UND BIOARTIFIZIELLES ORGAN MIT EINER DERARTIGEN MEMBRAN
FUNCTIONALIZED MEMBRANE FOR A CHAMBER FOR ENCAPSULATING CELLS PRODUCING AT LEAST ONE SUBSTANCE OF THERAPEUTIC INTEREST AND BIOARTIFICIAL ORGAN COMPRISING SUCH A MEMBRANE

(30) Priorité: 04.06.2010 FR 1002371
(43) Date de publication de la demande: 10.04.2013
(73) Titulaire: Association pour les transferts de technologie Du Mans, 72000 Le Mans (FR); Centre Européen d'Etude du Diabète, 67000 Strasbourg (FR)
(72) Inventeur: LEGEAY, Gilbert, F-72650 Saint Saturnin (FR); SIGRIST, Séverine, F-67230 Benfeld (FR); COUDREUSE, Arnaud, F-72100 Le Mans (FR)
(74) Mandataire: Bernstein, Claire Jacqueline
(86) Numéro de dépôt international: PCT/IB2011/052391
(87) Numéro de publication internationale: WO 2012/017337

(56) Documents cités:
- WO-A1-98/28026
- WO-A1-02/060409
- US-A1- 2005 152 950
- US-A1- 2006 198 864

## Description

La présente invention se rapporte au domaine des organes bioartificiels implantables dans l'organisme d'un patient et se présentant sous la forme de dispositifs encapsulant des cellules sécrétrices de substances d'intérêt thérapeutique.

Le traitement de pathologies nécessitant un apport continu à l'organisme en substances d'intérêt thérapeutique a rendu nécessaire la mise au point de dispositifs qui puissent être implantés chez un patient et capables de libérer ces substances de manière efficace parfois pendant de longues périodes de temps.

Pour répondre à ce besoin, il a été développé des organes bioartificiels qui renferment des cellules produisant une ou plusieurs substances d'intérêt thérapeutique. Les cellules contenues dans un organe bioartificiel sont confinées dans des espaces internes, ou chambres d'encapsulation, délimités par au moins une membrane semi-perméable. Une telle membrane semi-perméable doit permettre la diffusion des substances d'intérêt thérapeutique jusqu'aux cellules cibles dans le corps du patient, tout en étant imperméable aux anticorps et aux cellules du système immunitaire du patient.

Par organe bioartificiel, on entend un dispositif comprenant au moins une chambre d'encapsulation constituée d'au moins une membrane semi-perméable ; ladite chambre d'encapsulation est destinée à contenir des cellules sécrétrices d'une substance d'intérêt thérapeutique.

La substance d'intérêt thérapeutique peut être un neurotransmetteur, une hormone, un facteur de croissance ou une cytokine ; par exemple et sans caractère limitatif, l'insuline, l'hormone de croissance, la calcitonine.

Un exemple de ce type de dispositif est décrit dans la Demande Internationale WO 02/060409 qui vise plus particulièrement la mise au point de membranes semi-perméables aux propriétés mécaniques et de perméabilité sélective améliorées pour la fabrication d'organes bioartificiels consistant en une chambre d'encapsulation de cellules productrices d'une substance active. Implanté chez un patient, un tel organe bioartificiel permet la libération de la substance active et le traitement du patient.

Une difficulté rencontrée lors de l'implantation de ce type de dispositif est la relativement courte durée de son efficacité qui s'explique par un défaut d'oxygénation des cellules encapsulées dans la chambre d'encapsulation ainsi que leur inactivation par des cytokines de bas poids moléculaire produites par les cellules immunitaires du patient receveur.

Sigrist *et al.* ont amélioré la viabilité d'îlots pancréatiques encapsulés dans une chambre d'encapsulation constituée d'une membrane semi-perméable en polyacrylonitrileméthallylsulfonate de sodium (membrane « AN69 » de la société HOSPAL) en ajoutant, à l'intérieur de cette chambre, le facteur de croissance des cellules épithéliales (VEGF). Après implantation chez des souris d'un organe bioartificiel constitué de la chambre d'encapsulation ainsi préparée, la diffusion du VEGF dans les tissus environnant l'implant a permis l'induction de l'angiogénèse autour de l'implant (J. of Vascular Research, 2003, 40(4):359-67 et Cell Transplantation, 2003, vol.12, pp.627-635).

Cette technique présente toutefois des limites liées à la faible diffusibilité du VEGF au travers des parois de la chambre et à l'importance d'induire l'angiogénèse le plus tôt possible dès l'implantation de l'organe bioartificiel.

Le Brevet US 5,262,055 propose un pancréas artificiel constitué d'une chambre d'encapsulation équipée d'une membrane immunoprotective et qui contient des îlots pancréatiques dans une matrice polymère thermosensible ; le contenu de la chambre d'encapsulation peut être renouvelé après implantation grâce à deux tubes fins qui relient l'intérieur de la chambre d'encapsulation à l'extérieur de l'organisme du patient. Le caractère immunoprotecteur de la membrane semi-perméable est conféré par l'imperméabilité de la membrane qui empêche l'entrée dans le dispositif de cellules du système immunitaire. Ce brevet propose également l'inclusion, dans la matrice polymère thermosensible, de microparticules polymères permettant la libération d'agents actifs destinés à induire la vascularisation ou inhiber l'activité macrophagique autour de la chambre d'encapsulation. Le double inconvénient de ce dispositif est la localisation des microparticules polymères à l'intérieur de la chambre d'encapsulation (faible diffusibilité de la membrane semi-perméable) et la libération puis l'accumulation indésirable dans l'organisme du polymère constituant les microparticules lors de la libération des agents actifs.

La Demande Internationale WO 94/26399 décrit des membranes semi-perméables qui portent des agents actifs fixés par des liaisons covalentes ; ce type de membrane n'est toutefois pas satisfaisant car leur préparation demande une étape supplémentaire de fixation covalente des agents actifs et en ce que la quantité d'agents actifs est limitée à celle présente à la surface de la membrane.

La Demande US 2006/0198864 décrit des membranes pour interfaces biologiques destinées à recouvrir des dispositifs implantés tels que des sondes de mesure de la glycémie. Ces membranes ont une architecture en deux parties, la couche support et la couche externe qui présente un relief très marqué (architecture alvéolée présentant des cavités d'une taille de 20 à 1000 µm) et qui permet le développement de vaisseaux sanguins. Ce document décrit également la possibilité d'ajouter des agents actifs à ces membranes notamment pour limiter les manifestations inflammatoires et favoriser la vascularisation ; ces agents sont alors soit incorporés dans la matrice de la membrane (composée de polycarbonate, de PVA ou de polymères cellulosiques) soit adsorbés ou liés par des liaisons covalentes à la surface de la membrane. L'utilisation de ces membranes pour la fabrication de sondes destinées à la détection de substances telles que le glucose dans l'organisme nécessite un relief particulier et une grande perméabilité des membranes ; les inventeurs ont constaté que la structure particulière de ces membranes, en particulier, le fait que leurs pores soient interconnectés (ce qui conduit à l'obstruction des pores par des cellules empêchant ainsi la circulation des molécules biologiquement actives et des substances d'intérêt thérapeutique), que la couche support ne présente pas un seuil de coupure spécifiquement choisi pour assurer une perméabilité sélective et que les matériaux hydrophobes soient utilisés pour la fabrication de la couche externe de ces membranes, fait que ces supports ne sont pas compatibles avec leur utilisation pour la préparation d'un organe bioartificiel et pour son fonctionnement après implantation chez un individu.

Ainsi, plusieurs difficultés sont rencontrées lors de l'implantation d'un organe bioartificiel chez un patient receveur ; il faudra en premier lieu éviter ou limiter (i) la réaction inflammatoire des tissus du patient provoquée par la mise en place de l'organe bioartificiel et (ii) l'introduction dans la chambre réactionnelle de cytokines et de chimiokines qui détruiraient les cellules sécrétrices. En outre, l'oxygénation des cellules sécrétrices encapsulées dans l'organe bioartificiel et la diffusion optimale de la substance d'intérêt thérapeutique dans l'organisme nécessite la vascularisation des tissus environnant l'organe bioartificiel. Il demeure ainsi nécessaire d'améliorer les propriétés des membranes utilisées pour la fabrication d'organes bioartificiels afin d'accélérer l'implantation et la mise en fonctionnement (sécrétion des substances d'intérêt thérapeutique) desdits organes bioartificiels dans l'organisme des patients.

Les inventeurs ont mis au point un nouveau type de membranes semi-perméables fonctionnalisées par au moins deux molécules biologiquement actives ; lesdites membranes étant utiles pour la fabrication d'organe bioartificiel.

Une membrane semi-perméable est dite fonctionnalisée lorsqu'elle comporte une molécule biologiquement active dont la libération *in vivo* facilite l'implantation et améliore le fonctionnement de l'organe bioartificiel.

Les inventeurs ont montré que l'implantation d'organes bioartificiels composés d'une membrane semi-perméable fonctionnalisée par de l'héparine et du VEGF à titre de molécules biologiquement actives permettait d'éviter une réaction inflammatoire autour de l'organe bioartificiel et conduisait au développement de vaisseaux sanguins autour de l'organe bioartificiel au bout de seulement deux semaines ; une telle vascularisation n'étant observée qu'au bout de deux mois pour les organes bioartificiels dont les membranes semi-perméables ne sont pas fonctionnalisées.

La présente invention se rapporte ainsi à une membrane semi-perméable fonctionnalisée, composée d'un support biocompatible poreux préalablement traité pour en augmenter l'énergie de surface et caractérisée en ce qu'elle comprend également au moins deux couches comprenant chacune un polymère hydrophile et au moins une molécule biologiquement active.

Plus particulièrement, il s'agit d'une membrane semi-perméable fonctionnalisée composée d'un support biocompatible poreux caractérisée en ce que :
- ledit support biocompatible poreux est préalablement traité pour que son énergie de surface soit supérieure ou égale à 50 mJ.m⁻² ;
- les pores dudit support biocompatible poreux ont une taille interne comprise entre 5 et 100 nm et
- ladite membrane semi-perméable fonctionnalisée comprend au moins deux couches comprenant chacune un polymère hydrophile et au moins une molécule biologiquement active.

La préparation du support biocompatible peut être réalisée selon la méthode décrite dans la Demande Internationale WO 02/060409.

Le support biocompatible est constitué de polycarbonate ou de polyester ou de polyéthylénimine poreux ; son épaisseur est comprise entre 5 et 100 µm, de préférence, entre 10 et 60 µm. La formation des pores peut être réalisée par bombardement électronique ou par bombardement d'ions lourds ; cette seconde technique est notamment décrite dans le Brevet US 4,956,219. Dans le cas d'un bombardement d'ions lourds, la densité des ions lourds bombardés à la surface du support biocompatible détermine la densité des pores tandis que le temps de traitement d'érosion chimique détermine la taille des pores.

Dans le cadre de la présente invention, les pores réalisés sur le support biocompatible ont une taille interne comprise entre 5 et 100 nanomètres, de préférence entre 5 et 50 nanomètres.

La surface du support biocompatible poreux est ensuite traitée afin d'en augmenter l'énergie de surface. Le traitement du support conduit à la création de sites polaires à la surface du support biocompatible poreux, en particulier, le traitement provoque l'augmentation de la proportion des groupements carbonyle, hydroxy ou amine, et des radicaux libres. Les radicaux libres se recombinent entre eux, ou avec l'oxygène de l'air, créant ainsi des sites polaires.

On considère qu'une énergie de surface suffisante est telle que l'angle de contact mesuré entre la tangente d'une goutte d'eau posée sur le support et la surface de la membrane est inférieur à 40° ; ce qui correspond à une énergie de surface d'au moins 50 mJ.m⁻². Ainsi, l'objectif du traitement du support est d'atteindre une énergie de surface supérieure ou égale à 50 mJ.m⁻².

Les valeurs de mouillage et d'énergie de surface qui suivent sont données à titre d'exemple :
1) Support de polycarbonate non traité :
   Angle de mouillage (eau) : 60° ;
   Energie de surface : 41 à 44 mJ.m⁻² (composante polaire : 15 mJ.m⁻²)
2) Support de polycarbonate traité plasma :
   Angle de mouillage (eau) : 25° ;
   Energie de surface : 66 mJ.m⁻² (composante polaire : 37,2 mJ.m²)
3) Support de polycarbonate traité plasma et recouvert d'une couche hydrophile :
   Angle de mouillage (eau) : 20° ;
   Energie de surface : 66,7 mJ.m⁻² (composante polaire : 40 mJ.m²).

En particulier, dans le cas d'un support biocompatible en polycarbonate, les sites polaires présents à la surface du support biocompatible constitutif de la membrane semi-perméable de l'invention comprennent les sites suivants : CH₃O, C₂H₃O, C₃H₃O, C₃H₇O, O, OH, C₂OH, C₈H₅O, NH₄⁺, C₂H₈N⁺, R-OH (alcool), (R)₃-NH (amine) et R-CO-NH (amide), dans lesquels le substituant R représente un radical constitutif du polymère de polycarbonate du support biocompatible. Ces sites polaires augmentent l'énergie de surface du support biocompatible et permettent ainsi l'adhérence des couches de polymère hydrophile.

De manière préférentielle, la création de sites polaires à la surface du support biocompatible est réalisée par un traitement par plasma, par décharges couronnes ou encore par décharges électromagnétiques à pression atmosphérique ou sous vide.

Par exemple, le support est traité par un plasma radiofréquence d'argon. Il peut être traité à une puissance d'émission du réacteur à plasma comprise entre 2 et 10 watts par litre de capacité du réacteur, entre 1 et 30 minutes. Le traitement peut aussi être effectué par un plasma micro-ondes, à la même puissance, mais durant 5 secondes à 20 minutes.

De manière préférée, le traitement par plasma est effectué sous vide.

Pour la mise en oeuvre d'un procédé de traitement par plasma, l'homme du métier pourra avantageusement se référer à l'ouvrage de A. Ricard (« Plasmas réactifs », Editions SVF, 1995).

Le traitement peut aussi être effectué par décharges couronnes. La tension de traitement est avantageusement comprise entre 50 et 500 volts, l'intensité étant variable selon le dispositif de traitement et les supports traités.

Le traitement par décharges couronnes peut être effectué à l'aide de dispositifs à électrodes parallèles en vis-à-vis, à électrodes parallèles côte à côte (arc électrode d'environ 5 mm de hauteur), ou à arc soufflé (électrodes parallèles côte à côte avec courant gazeux entre elles, créant ainsi un arc électrique d'environ 10 cm de hauteur).

Pour la réalisation d'un procédé de traitement par décharges couronnes ou par décharges électromagnétiques, l'homme du métier pourra avantageusement se référer à l'ouvrage d'A. Ricard (1995).

La durée du traitement est de l'ordre de quelques dixièmes de seconde, préférentiellement compris entre 0,1 et 1 seconde. En cas de traitement en continu, la durée d'exposition est telle que le matériau à traiter passe à travers le dispositif de traitement à une vitesse de quelques centimètres à plusieurs décimètres par seconde.

En outre, le support biocompatible peut être traité à plusieurs reprises afin d'augmenter l'efficacité du traitement.

De manière tout à fait préférée et dans le cas d'un support biocompatible en polycarbonate, la création de sites polaires est réalisée par une étape de traitement par plasma d'argon dans un réacteur de type Branson RF de 20 litres effectuée à la puissance de 50 watts pendant dix minutes. Dans ce mode de réalisation particulier, il a été observé à la surface du support biocompatible de polycarbonate, par mesure de spectrométrie de masse des ions secondaires, la composition suivante, en sites polaires quantifiée par l'intensité des ions secondaires détectés aux rapports masse/charge (m/z) suivants :
- en mode positif : 31 (CH₃O), 43 (C₂H₃O), 55 (C₃H₃O), 59 (C₃H₇O), 18 (NH₄) et 46 (C₂H₈N⁺) ; et
- dans le mode négatif : 16 (O), 17 (OH), 41 (C₂OH), 117 (C₈H₅O).

Selon l'invention, chaque couche de polymère hydrophile comprenant au moins une molécule biologiquement active a une épaisseur comprise entre 10 et 1000 nanomètres, préférentiellement entre 10 et 100 nanomètres et de manière tout à fait préférée entre 10 et 50 nanomètres.

Dans le cadre de la présente invention, on entend par polymère hydrophile, un polymère qui, lorsqu'il est appliqué sur un support biocompatible poreux est tel qu'une goutte d'eau déposée sur ledit polymère présente une valeur d'angle inférieure à 25°, de préférence inférieure à 22° après mesure selon le test de la « goutte sessile » tel que décrit dans la Demande Internationale WO 02/060409.

Préférentiellement, le polymère hydrophile est soluble dans l'eau. En effet, du fait de l'implantation de l'organe bioartificiel dans le corps d'un organisme hôte, l'utilisation de solvants organiques n'est pas recommandée car leur élimination totale est difficile, et leur présence, même en faibles quantités, n'est pas compatible avec une utilisation thérapeutique ou chirurgicale chez l'homme ou l'animal.

Le polymère hydrophile est de préférence choisi parmi les polymères suivants :
- les composés cellulosiques, tels que l'éthylcellulose (EC), l'hydroxypropyl méthylcellulose (HPMC), par exemple l'HPMC E4M commercialisée par la Société DOW CHEMICALS, ou celle dénommée Aqualon commercialisée par la Société Herculès, ou encore la carboxyméthylcellulose (CMC) commercialisée par la Société Herculès ;
- les polyacrylamides et leurs copolymères, tels que ceux commercialisés par la Société SIGMA (UPSALA, Suède) :
- la polyvinylpyrrolidone (PVP) et ses copolymères, tels que ceux commercialisés par la Société BASF, comme les Kollidon (K30, K90) ;
- les polyvinylalcools ;
- les copolymères de l'acétate de vinyle, tel que le copolymère de polyacétate de vinyle et d'alcool polyvinylique commercialisé sous le nom de Mowiol par la Société HOECHST ;
- les polyéthylène glycols, tels que ceux commercialisés par la Société SIGMA ;
- les polypropylène glycols ;
- les poly(méth)acrylates hydrophiles, tels que ceux commercialisés par les Sociétés DEGALAN ou DEGUSSA ;
- les polysaccharides ;
- les polymères à base d'acide hyaluronique ;
- les chitosans, tels que ceux commercialisés par la Société SIGMA.

De préférence, le polymère hydrophile est choisi parmi les composés cellulosiques, notamment l'HPMC, l'EC ou la CMC, les polyvinylpyrrolidones, les polyvinylalcools et certains polyacrylates tels que le poly(hydroxyéthyl acrylate) (HEMA) ou des copolymères d'acide acrylique.

Le polymère hydrophile utilisable selon l'invention pourra également être composé d'un mélange de deux ou plusieurs polymères hydrophiles mentionnés ci-dessus, par exemple, HPMC et CMC, HPMC et EC, ...

La molécule biologiquement active est mélangée au polymère hydrophile et destinée à être libérée dans le milieu environnant la membrane semi-perméable afin d'induire une réponse du ou des tissus du patient receveur de l'organe bioartificiel.

La réponse du tissu qu'on souhaite induire peut être de plusieurs natures ; en général, les molécules biologiquement actives qui pourront être introduites dans les membranes semi-perméables fonctionnalisées selon l'invention sont choisies parmi les agents anti-inflammatoires, les agents anti-infectieux, les anesthésiques, des facteurs de croissance, les agents stimulants l'angiogenèse et/ou induisant la vascularisation, les agents inducteurs de la cicatrisation, les agents immunosuppresseurs, les agents anti-thrombotiques incluant les agents antiagrégants et les agents anticoagulants, les inhibiteurs de l'enzyme de conversion de l'angiotensine (ECA), ou encore les molécules stimulant l'insulino-sécrétion (l'IGF, le glucagon-like peptide 1 (GLP-1) ou ses dérivés, les mimétiques des incrétines).

Parmi les agents anti-inflammatoires, on peut citer les anti-inflammatoires non stéroïdiens (AINS) tels que l'acétaminophène, l'acide aminosalicylique, l'aspirine, le célécoxib, le choline magnésium trisalicylate, le déclofénac, le diflunisal, l'étodolac, le flurbiprofène, l'ibuprofène, l'indométacine, l'interleukine IL-10, IL-6 mutéine, anti-IL-6, les inhibiteurs de NO synthase (par exemple, le L-NAME ou de L-NMDA), l'interféron, le kétoprofène, le kétorolac, le léflunomide, l'acide méfénamique, l'acide mycophénolique, la mizoribine, la nabumétone, le naproxène, l'oxaprozine, le piroxicam, le rofécoxib, le salsalate, le sulindac, et la tolmétine et les corticoïdes comme la cortisone, l'hydrocortisone, la méthylprednisolone, la prednisone, la prednisolone, le bétaméthasone, le dipropionate de bétaméthasone, le valérate de bétaméthasone, le dipropionate de béclométhasone, le budésonide, le phosphate sodique de dexaméthasone, le flunisolide, le propionate de fluticasone, le paclitaxel, le tacrolimus, le tranilast, l'acétonide de triamcinolone, l'acétonide de fluocinolone, le fluocinonide, le désonide, la désoximétasone, la fluocinolone, la triamcinolone, la triamcinolone, le propionate de clobétasol, et la dexaméthasone.

On préfère utiliser des agents antithrombotiques tels que les antiagrégants (l'acide acétylsalicylique, le clopidogrel, le ticlopidine, le dipyridamole, l'abciximab, l'eptifibatide et le tirofiban), les anticoagulants (l'héparine, la bivalirudine, le dabigatran, la lépirudine, le fondaparinux, le rivaroxaban, l'époprosténol, la warfarine, la phenprocoumone, la protéine C, la drotrécogine alfa, l'antithrombine, le pentosan) et les thrombolytiques (l'altéplase, l'urokinase, la ténectéplase et la rétéplase).

L'agent antithrombotique préféré est une héparine.

En outre, il est avantageux d'utiliser, à titre de molécule biologiquement active, une molécule permettant une vascularisation satisfaisante des tissus environnant l'organe bioartificiel, il peut notamment s'agir de facteurs de croissance tels que :
- *ceux de la famille des PDGF (Platelet derived growth factor) :* PDGF1, PDGF2, VEGF (facteur de croissance vasculaire), VPF (facteur de perméabilité vasculaire) ;
- *ceux de la famille de l'EGF (Epidermal Growth Factor) :* EGF, l'urogastrone (URO), le TGFα (Transforming Growth Factor α), l'amphiréguline ;
- *ceux de la famille des FGF (Fibroblast Growth Factor) :* numérotés de 1 à 6, et pouvant être complexés par l'héparine ;
- *ceux de la famille de l'insuline :* IGF-1 et IGF-2 ;
- *les facteurs neurotropes* (NGF) ;
- *le facteur de croissance du tissu conjonctif* (CTGF) ;
- *les facteurs de croissance des hépatocytes* (HGF).

De préférence il s'agit de facteurs de croissance cellulaire qui favorisent la vascularisation par l'induction de l'angiogénèse tels que le facteur de croissance basique des fibroblastes (bFGF), le facteur de croissance de l'endothélium vasculaire (VEGF), le facteur de croissance des cellules endothéliales dérivé des plaquettes (PDGF1 ou 2) ou le facteur de croissance des hépatocytes (HGF).

Pour la préparation de la couche de polymère hydrophile et de molécule biologiquement active, le polymère hydrophile ou le mélange de polymères hydrophiles est mis en solution dans de l'eau.

Quel que soit le polymère hydrophile, ou le mélange de polymères hydrophiles, utilisé, sa quantité en poids total de la solution est de préférence ajustée de manière à obtenir une solution aqueuse de polymère hydrophile ayant une viscosité comprise entre 1 et 10 centipoises.

Par exemple, une valeur de viscosité de l'ordre de 5 à 10 centipoises (cPs) est obtenue pour une solution aqueuse ayant une concentration de 1% en poids de PVP (Kollidon K90 commercialisé par la Société BASF) ou encore pour une solution aqueuse ayant une concentration de 0,2% en poids de HPMC (E4M commercialisée par DOW CHEMICALS). Les mesures de viscosité sont réalisées à l'aide d'une aiguille de type DIN 30D, à température ambiante et pour une vitesse de rotation de 300 à 500 t/min.

Ainsi, et à titre d'illustration, lorsque le polymère hydrophile est de l'hydroxypropyl méthylcellulose (HPMC), de la polyvinylpyrrolidone (PVP) ou encore un mélange de ces deux polymères, le pourcentage en poids du polymère hydrophile, par rapport au poids total de la solution aqueuse de polymère, est avantageusement compris entre 0,1% et 1%.

La molécule biologiquement active est ensuite ajoutée à cette solution aqueuse de polymère hydrophile puis solubilisée ou mise en suspension par agitation avec un ratio molécule biologiquement active / polymère hydrophile compris entre 25/100 et 75/100 en poids, de préférence, ce ratio est compris entre 25/100 et 50/100, le ratio est préférentiellement de 25/100 quels que soient la molécule biologiquement active et le polymère hydrophile.

L'application des couches du polymère hydrophile et de la molécule bioactive sur le support biocompatible peut être réalisée par trempage.

De manière tout à fait préférée, la durée de l'étape du trempage est comprise entre 5 secondes et 10 minutes.

Avantageusement, l'étape de trempage a lieu à une température comprise entre 15°C et 25°C.

La durée de l'étape de trempage du support biocompatible dans une solution de polymère hydrophile est ajustée de telle manière à obtenir une couche de polymère d'une épaisseur comprise entre 10 et 1000 nanomètres, préférentiellement entre 10 et 100 nanomètres et de manière tout à fait préférée entre 10 et 50 nanomètres.

Chacune des couches de polymère hydrophile et de molécule biologiquement active suivantes est réalisée selon ce même procédé après séchage complet de la couche précédente. Il est aussi possible de réaliser un empilement successif de couches fonctionnalisées pour augmenter les quantités de molécules biologiquement actives ; le nombre de couches fonctionnalisées peut être compris entre 2 et 10, de préférence entre 2 et 5.

Selon une variante préférée de mise en oeuvre de l'invention, deux couches de polymère hydrophile et de molécule biologiquement active sont appliquées sur le support biocompatible, la première couche (positionnée entre le support et la seconde couche) comprend un facteur de croissance cellulaire, par exemple, du VEGF dans de l'EC, et la seconde couche (externe) comprend un agent antithrombotique, par exemple, une héparine dans l'HPMC ; il s'agit là d'une variante préférée, l'empilement de ces deux couches fonctionnalisées pourrait être inversé (c'est-à-dire, une première couche comprenant le VEGF dans l'HPMC et une seconde couche comprenant une héparine dans l'EC).

De façon avantageuse, les membranes semi-perméables de l'invention présentent de très bonnes propriétés de perméabilité aux substances d'intérêt thérapeutique produites à l'intérieur des organes bioartificiels et une bonne perméabilité aux nutriments de l'organisme nécessaires à la viabilité des cellules contenues dans l'organe bioartificiel.

De façon également très avantageuse, les cellules adhèrent très faiblement à la surface des membranes semi-perméables selon l'invention.

En résumé, les membranes semi-perméables fonctionnalisées selon l'invention présentent les caractéristiques suivantes :
- elles possèdent un seuil de coupure compris entre 10.000 et 50.000 Daltons, de préférence entre 10.000 et 30.000 Daltons, et de manière tout à fait préférée entre 10.000 et 15.000 Daltons ;
- elles possèdent une densité de pores comprise entre 10⁹ et 10¹¹ pores/cm² ;
- elles possèdent une épaisseur comprise entre 5 µm et 100 µm, de préférence entre 10 µm et 60 µm.

Selon un premier mode de réalisation, les couches de polymères hydrophiles et de molécules biologiquement actives peuvent recouvrir les deux faces du support biocompatible.

Dans un second mode préféré de réalisation, les couches de polymères hydrophiles et de molécules biologiquement actives ne recouvrent qu'une seule des deux faces du support biocompatible ; selon ce mode de réalisation, la face du support biocompatible recouverte de polymères hydrophiles et de molécules biologiquement actives est celle située du côté externe de l'organe bioartificiel et qui est en contact avec le milieu environnant le lieu d'implantation de cet organe.

L'invention a également pour objet l'utilisation d'une membrane fonctionnalisée selon l'invention pour la fabrication d'une chambre d'encapsulation de cellules sécrétrices produisant au moins une substance d'intérêt thérapeutique

L'invention a encore pour objet une chambre d'encapsulation de cellules sécrétrices produisant au moins une substance d'intérêt thérapeutique caractérisée en ce que ses parois sont constituées d'une membrane semi-perméable fonctionnalisée telle que définie ci-dessus délimitant un espace susceptible de contenir les cellules sécrétrices produisant au moins une substance d'intérêt thérapeutique et un organe bioartificiel comprenant une chambre d'encapsulation de cellules sécrétrices produisant au moins une substance d'intérêt thérapeutique caractérisée en ce que les parois de la chambre d'encapsulation sont constituées d'une membrane semi-perméable fonctionnalisée telle que définie ci-dessus.

L'utilisation des membranes semi-perméables selon l'invention pour la fabrication d'organes bioartificiels conduit à des dispositifs dont l'implantation est favorisée par la libération d'anti-thrombotiques et la constitution de vaisseaux sanguins autour dudit dispositif lors de son implantation.

Une chambre d'encapsulation selon l'invention peut avoir les caractéristiques des chambres constitutives d'organes bioartificiels décrits dans l'état de la technique.

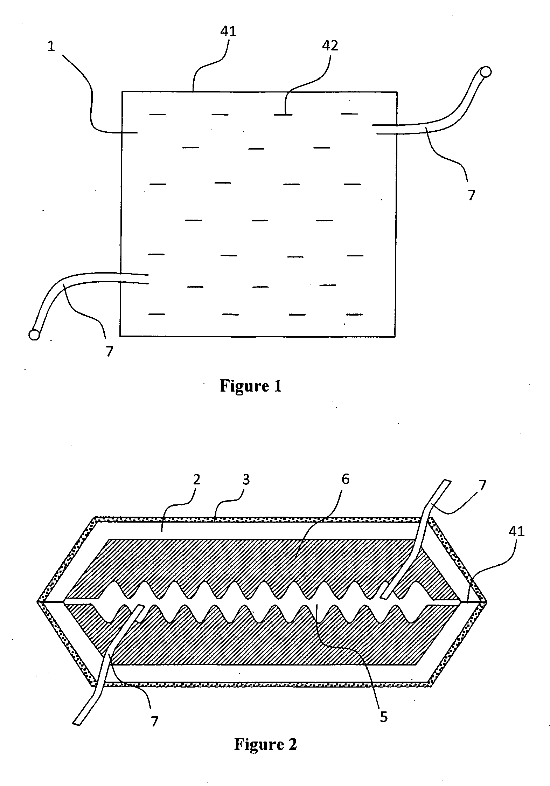

Un exemple non limitatif d'un mode de réalisation d'un organe bioartificiel comprenant une chambre d'encapsulation selon l'invention, un tel organe bioartificiel est illustré aux Figures 1 (vue de dessus) et 2 (coupe transversale).

Selon ce mode de réalisation particulier, la chambre d'encapsulation (1) est de forme parallélépipédique et comprend deux membranes semi-perméables fonctionnalisées, une supérieure et une inférieure, constituées d'un support biocompatible (2) sur lequel sont positionnées des couches de polymère hydrophile et de molécule biologiquement active (3) ; ces deux membranes semi-perméables sont soudées sur leur bord externe (41) et sur différents points de leur surface (42).

Les deux membranes fonctionnalisées délimitent l'enceinte (5) de la chambre d'encapsulation dans laquelle sont contenues des cellules produisant au moins une substance d'intérêt thérapeutique. L'enceinte est avantageusement garnie d'une matrice interne (6). La hauteur de l'intérieur de la chambre d'encapsulation correspond à la hauteur des cellules ou des groupes de cellules sécrétrices (par exemple dans le cas des îlots pancréatiques) ; à titre d'exemple, cette hauteur sera comprise entre 5 et 900 µm).

Les points de soudure (42) positionnés sur la surface des membranes permettent une meilleure répartition des cellules sécrétrices lors du remplissage de la chambre d'encapsulation et, en particulier, d'éviter que les cellules sécrétrices s'agrègent.

Enfin, selon une variante préférée, l'organe bioartificiel est en outre équipé de deux tuyaux flexibles (7) permettant de remplir et vider l'organe bioartificiel ; ceci revêt un avantage particulier pour renouveler le contenu de l'organe bioartificiel lorsqu'il est implanté chez un patient, sans procéder à une explantation.

Les cellules produisant au moins une substance d'intérêt thérapeutique peuvent être, par exemple, des cellules des îlots pancréatiques (ou îlots de Langherans) ou des cellules à insuline provenant de lignées cellulaires comme MIN-6, RIN-m5f et INS-1, qui produisent de l'insuline lorsque la chambre d'encapsulation est destinée à la fabrication d'un pancréas bioartificiel.

Les cellules peuvent aussi être des cellules hépatiques lorsque la chambre d'encapsulation est destinée à la fabrication d'un foie bioartificiel.

Dans un mode de réalisation particulier, les cellules sont transfectées ou transformées par au moins un acide nucléique permettant l'expression d'une substance d'intérêt thérapeutique. Parmi les substances d'intérêt thérapeutique, on peut citer, à titre illustratif, l'insuline, les cytokines, les hormones peptidiques, l'hormone de croissance et la calcitonine.

De manière générale, on entend par substance d'intérêt thérapeutique au sens de l'invention, une substance qui est libérée ou sécrétée par la cellule qui la produit et exerce son effet sur une cellule cible ou sur une molécule cible dans l'organisme hôte, comme par exemple un neurotransmetteur, une hormone, un facteur de croissance ou une cytokine.

Une grande diversité de cellules peut être utilisée, incluant des lignées cellulaires immortalisées comme des cultures primaires de cellules en division.

Les cellules peuvent être par exemple des myoblastes, qui sont des cellules précurseurs des cellules musculaires dérivées des populations de cellules souches du mésoderme, et qui peuvent être facilement transformées par un acide nucléique permettant l'expression de la substance d'intérêt thérapeutique. L'homme du métier pourra avantageusement se référer par exemple aux demandes PCT publiées sous les numéros WO 94/02129, WO 93/03768 et WO 90/15863.

Les cellules peuvent aussi être des cellules bêta des îlots de Langherans du pancréas ou encore des hépatocytes, de préférence d'origine humaine.

Les cellules contenues dans une chambre d'encapsulation selon l'invention peuvent être incluses dans une matrice, telle qu'une matrice de collagène de type IV, le cas échéant en association avec de la laminine, de l'entactine et de l'héparane sulfate comme la matrice commercialisée sous le nom de Matrigel.

Les cellules contenues dans une chambre d'encapsulation selon l'invention peuvent, de manière générale, être incluses dans une matrice composée de tout produit ou combinaison de produits permettant l'immobilisation de ces cellules sous une forme viable.

Selon un autre aspect, l'invention est également relative à un organe bioartificiel caractérisé en ce qu'il comprend au moins une chambre d'encapsulation telle que définie ci-dessus.

Les caractéristiques d'un organe bioartificiel selon l'invention peuvent être de toute nature connue en soi dans l'état de la technique.

Pour la réalisation d'un organe bioartificiel de l'invention, dont la caractéristique essentielle est de comprendre au moins une chambre d'encapsulation de cellules, dotée d'une membrane semi-perméable fonctionnalisée selon l'invention, l'homme du métier pourra avantageusement se référer aux brevets US 5,981,211, US 4,578,191, US 5,837,234, US 6,023,009, US 5,605,835 et US 4,323,457.

Selon un mode de réalisation particulier de l'invention, l'organe bioartificiel est un pancréas bioartificiel contenant des cellules des îlots de Langherans.

Selon un second mode particulier de réalisation de l'invention, l'organe bioartificiel est un foie artificiel contenant des cellules hépatiques.

A titre illustratif, un organe bioartificiel selon l'invention peut être implanté de manière intra-péritonéale ou encore au-dessus de la capsule rénale ; il peut rester implanté au moins cinq ans, si nécessaire, son contenu (cellules sécrétrices) peut être renouvelé.

### Figures

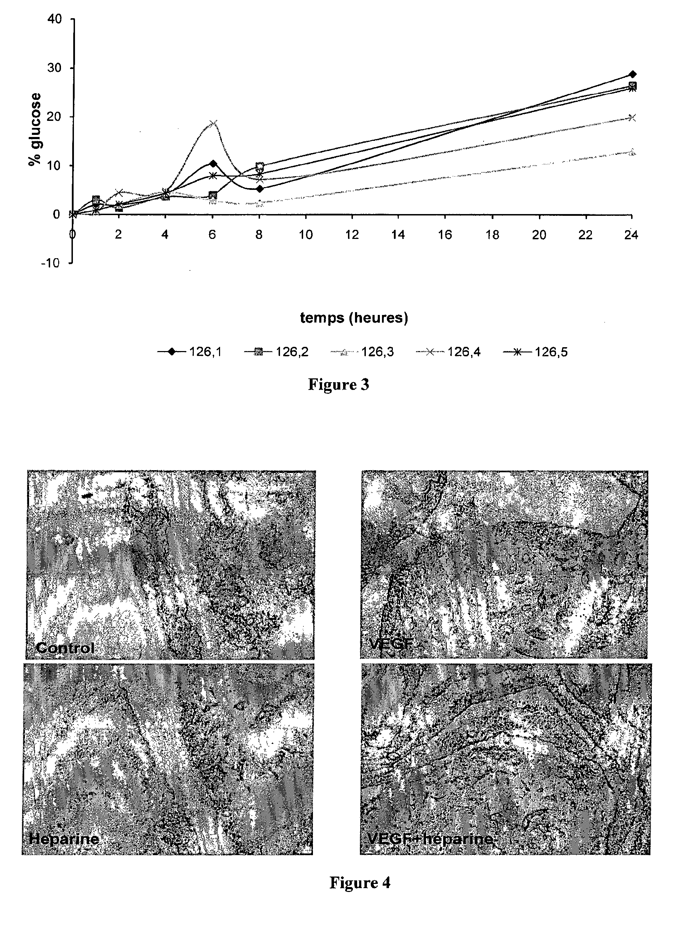

La **Figure 1** représente la vue de dessus d'un exemple de mode de réalisation d'un organe bioartificiel selon l'invention, la **Figure 2** représente une coupe transversale de ce même organe bioartificiel ; cet organe bioartificiel comprend une chambre d'encapsulation (1) de forme parallélépipédique et deux membranes semi-perméables fonctionnalisées, une supérieure et une inférieure, constituées d'un support biocompatible (2) sur lequel sont positionnées des couches de polymères hydrophiles et de molécules biologiquement actives (3) ; ces deux membranes semi-perméables sont soudées sur leur bord externe (41) et sur différents points de leur surface (42) ; les deux membranes fonctionnalisées délimitent l'enceinte (5) de la chambre d'encapsulation dans laquelle sont contenues des cellules produisant au moins une substance d'intérêt thérapeutique ; l'enceinte est garnie d'une matrice interne (6).
Le graphe de la **Figure 3** représente la perméabilité au glucose des membranes préparées dans l'exemple 1.

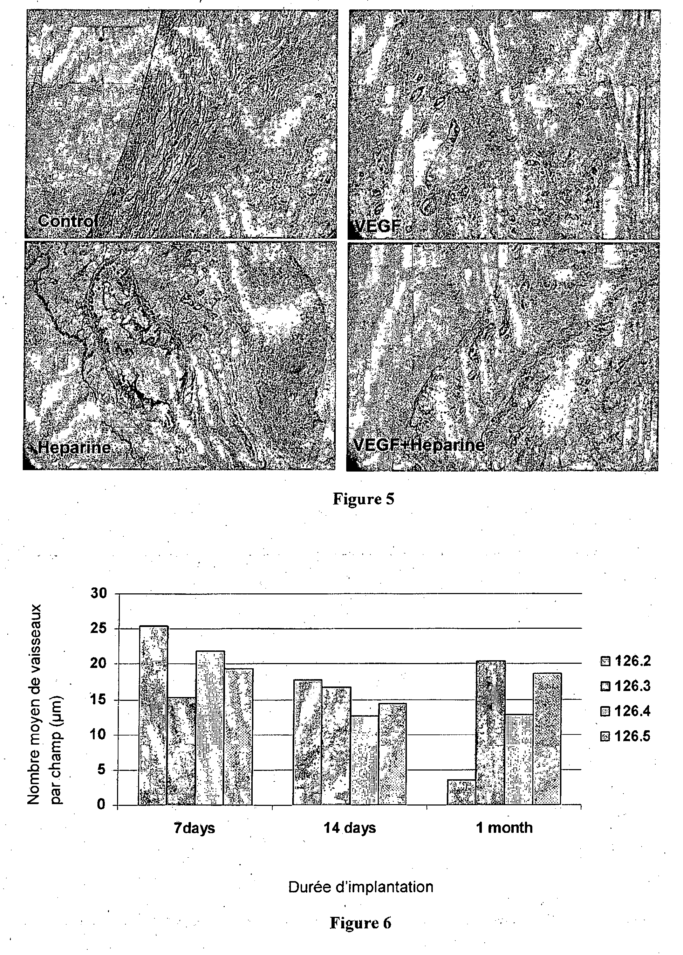

Les **Figures 4** et **5** sont des clichés de coupes histologiques du tissu épiploïque environnant la membrane : sans molécule biologiquement active, avec de l'héparine, avec du VEGF et avec de l'héparine et du VEGF, après 7 jours **(****Figure 4****)** ou 1 mois **(****Figure 5****)** chez des rats (x400).

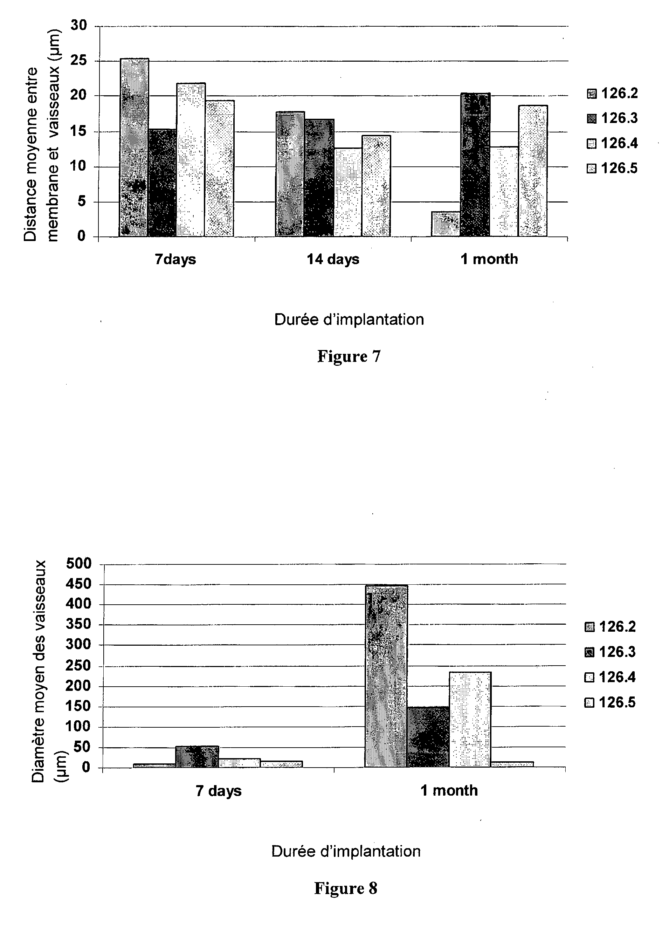

Sur la base des clichés réalisés après 7 jours, 14 jours et 1 mois d'implantation, plusieurs paramètres caractérisant l'évolution de la vascularisation des tissus environnant l'implant ont été évalués, il s'agit de :
- la moyenne du nombre de vaisseaux sanguins par champs dénombrés dans 5 champs des coupes après 7 jours, 14 jours ou 1 mois d'implantation pour les différentes membranes testées **(****Figure 6****) ;**
- la distance moyenne entre la membrane semi-perméable et les premiers vaisseaux sanguins dénombrés dans 5 champs des coupes après 7 jours ou 1 mois d'implantation pour les différentes membranes testées **(****Figure 7****) ;**
- le diamètre moyen des vaisseaux sanguins mesuré dans 5 champs des coupes après 7 jours ou 1 mois d'implantation pour les différentes membranes testées **(****Figure 8****).**

### Exemples

### Exemple 1 - Fabrication de membranes semi-perméables

### I. Membranes comprenant un support en polycarbonate recouvert d'hydroxypropyl méthylcellulose (HPMC) seul (membranes contrôles)

### I.A Préparation des solutions d'HPMC

Les solutions suivantes sont préparées au moins la veille du traitement par la dissolution de 2 g d'HPMC (E4M de Dow Chemical) dans 1 l d'eau PPI ou un ratio équivalent pour obtenir une concentration de 0,2% d'HPMC. La solution est laissée une nuit sous agitation (agitateur magnétique) à température ambiante.

### I.B Traitement plasma des supports de polycarbonate et enduction

Les membranes support en polycarbonate Pokalon (Lonza) sont préalablement nettoyées à l'eau PPI.

Les traitements des membranes support de polycarbonate sont réalisés en salle blanche avec une machine plasma Branson ; le traitement suivant est appliqué : Ar - 50 W - 10 minutes.

Les membranes supports de polycarbonate ainsi traitées sont trempées immédiatement dans la solution d'HPMC puis sont mises à sécher à 45°C à l'étuve.

### II. Membranes fonctionnalisées avec du VEGF dans de l'HPMC selon l'invention

### II.A Préparation des solutions d'HPMC et de VEGF

Une solution d'HPMC à 0,1% (1 g/l) est préparée dans de l'eau PPI.

1 ml de cette solution d'HPMC est diluée dans 100 ml de tampon phosphate DPBS conduisant à une concentration en HPMC de 0,001%.

La préparation d'une solution de VEGF (Tebu-bio SAS) à 10 µg/ml (soit 10 mg/l) est réalisée par l'ajout de 1 ml de DPBS dans le vial contenant 10 µg de VEGF.

L'ensemble de cette solution de VEGF (soit 1 ml comprenant donc 10 µg de VEGF) est mélangé avec 4 ml de solution d'HPMC (comprenant 40 µg d'HPMC) pour préparer une solution telle qu'elle comprend 25 parts de VEGF pour 100 parts d'HPMC.

Le mélange est introduit dans une fiole de 50 ml et dilué afin d'obtenir des concentrations finales de 0,2 µg/ml pour le VEGF et de 0,8 µg/ml pour l'HPMC.

Selon le même principe, des solutions telles qu'elles comprennent les ratios HPMC/VEGF de 100/50 et de 100/10 sont préparées.

### II.B Traitement plasma et enduction

Ces étapes sont réalisées comme décrit au point I.B à l'exception du séchage qui est réalisé à température ambiante.

### III. Membranes fonctionnalisées avec de l'héparine dans de l'éthyl cellulose (EC) avec un ratio EC/héparine de 100/25 selon l'invention

### III.A Préparation des solutions

La solution d'EC est préparée comme suit : l'EC se présente sous la forme d'une pâte (Surelease de Colorcon, à 25% soit 25mg/100mg), elle est diluée dans de l'eau PPI à 1 g/l soit 250 mg/l d'EC.

Cette solution est diluée une seconde fois dans l'eau PPI pour conduire à une solution à 10 mg/l d'EC.

La solution commerciale d'héparine (héparine Choay 25000UI Sanofi-Aventis) a une concentration en héparine de 200mg/5ml ; elle est diluée dans de l'eau PPI jusqu'à atteindre une concentration en héparine de 10 µg/ml.

1 ml de la solution d'héparine (contenant 10 µg d'héparine) est mélangé à 4 ml de la solution d'EC (contenant 40 µg d'EC) vont conduire à une solution présentant un ratio de 25 parts d'héparine pour 100 parts d'EC.

Cette solution est introduite dans une fiole de 50 ml et diluée afin de ramener les concentrations à 0,2 µg/ml pour l'héparine et à 0,8 µg/ml pour l'EC.

### III.B Traitement plasma et enduction

Ces étapes sont réalisées comme décrit au point I.B à l'exception du séchage qui est réalisé à température ambiante.

### IV. Membranes fonctionnalisées avec de l'héparine dans de l'EC et du VEGF dans de l'HMPC selon l'invention

### IV.A Préparation des solutions

Les solutions d'héparine dans l'EC et de VEGF dans l'HPMC sont préparées comme indiquées respectivement aux points III.A. et II.A.

### IV.B Traitement plasma et enduction

Le traitement plasma est réalisé comme décrit au point I.B.

Les enductions sont réalisées dans l'ordre suivant :
- trempage du support de polycarbonate dans la solution d'EC/héparine puis séchage à température ambiante ;
- puis trempage du support fonctionnalisé avec l'héparine dans la solution d'HPMC/VEGF puis séchage à température ambiante.

### V. Préparation du dispositif selon l'invention avec un support en silicone

Des dispositifs selon l'invention peuvent être préparés avec un support en silicone. Ces supports constituent une partie complémentaire des membranes semi-perméables selon l'invention.

Les supports en silicone (Nusil) sont préalablement traités par plasma dans les conditions suivantes : Ar - 100 W - 7,5 minutes.

Ensuite, l'application des couches fonctionnalisées est réalisée comme décrit dans les protocoles des parties II, III et IV ; l'eau PPI pouvant être remplacée par de l'eau distillée.

### Exemple 2 - caractérisation de la perméabilité des membranes semi-perméables

Des essais de perméabilité au glucose et aux immunoglobulines (IgG) des membranes préalablement préparées sont mis en oeuvre comme suit :
**Matériel :** chambre de diffusion constituée d'un compartiment haut et d'un compartiment bas séparés par une membrane dont on veut tester la perméabilité (l'étanchéité entre les deux compartiments est assurée par un joint), glucose (Prolabo), NaCl (Sigma, ref. S3014), IgG (Sigma, ref. I96640), eau distillée.

### Préparation des solutions

- *sérum physiologique, pour 1 litre* :

| | |
|---|---|
| NaCl | 9 g |
| Eau distillée | 1 l |

- *solution de glucose, pour 1 litre :*

| | |
|---|---|
| Glucose | 4 g |
| Sérum physiologique | 1 l |

- *solution d'IgG (concentration finale 5,75 µg*/*ml), pour 100 ml :*

| | |
|---|---|
| IgG mère (10 mg/ml) | 575 µl |
| Sérum physiologique | 99,425 ml |

### Protocole :

3 ml de sérum physiologique sont introduits dans le compartiment bas de la chambre de diffusion, la membrane dont on veut tester la perméabilité est positionnée au dessus du sérum physiologique en évitant la présence de bulles d'air.

3 ml de solution de glucose sont introduits dans le compartiment haut, la chambre de diffusion est fermée avec du parafilm puis est incubée à 37°C.

Pour le glucose : 1 ml de la solution se trouvant dans le compartiment haut de la chambre de diffusion est prélevé après homogénéisation douce. La membrane est ensuite retirée et mise dans une boîte de Pétri et 1 ml de la solution du compartiment bas est prélevé après homogénéisation.

Le dosage enzymatique du glucose est réalisé à l'aide du kit Glucose RTU (BioMérieux, référence 61 269).

Les résultats (exprimés en teneur en glucose dans le compartiment bas en fonction du temps) sont présentés dans le graphe de la **Figure 3****.**

La comparaison de la perméabilité vis-à-vis du glucose des membranes fonctionnalisées selon l'invention (126.3, 126.4 et 126.5) par rapport aux membranes de l'état de la technique dont la préparation est décrite dans l'exemple 1, point I (126.1 et 12.6.2) démontre que le traitement destiné à fonctionnaliser les membranes ne réduit pas la perméabilité au glucose.

Pour les IgG : 1 ml de la solution se trouvant dans le compartiment haut de la chambre de diffusion est prélevé après homogénéisation douce. La membrane est ensuite retirée et mise dans une boîte de Pétri où elle est rincée avec 1 ml d'eau distillée, l'eau de rinçage est conservée. 1 ml de la solution du compartiment bas est prélevé après homogénéisation.

Le dosage des IgG est réalisé par la méthode Bradford (Bradford, 1976 & Wright *et al.,* 1996).

Les membranes semi-perméables selon l'invention sont totalement imperméables aux IgG ; elles empêchent ainsi avantageusement la pénétration dans la chambre d'encapsulation de cytokines et chimiokines.

### Exemple 3 - essais d'implantation des membranes semi-perméables

Les membranes préparées comme décrit dans l'Exemple 1 sont implantées dans la cavité péritonéale de rat Wistar sain.

Dans la suite de la partie expérimentale, les membranes sont codées comme suit :
- 126.1 et 126.2 : membranes « contrôle » (préparées comme décrit dans la partie I de l'exemple 1) ;
- 126.3 : membrane « VEGF / HPMC » (ratio : 25/100, préparée comme décrit dans la partie II de l'exemple 1) ;
- 126.4 : membrane « héparine / EC » (ratio : 25/100, préparée comme décrit dans la partie III de l'exemple 1) ;
- 126.5 : membrane « VEGF / HMPC + héparine / EC » (pour les deux couches, le ratio est de 25/100, la membrane est préparée comme décrit dans la partie IV de l'exemple 1).

Des rats Wistar mâles de 230-250 g sont anesthésiés au gaz (isoflurane). Une laparotomie de 3 cm au niveau de la fosse iliaque gauche permet l'implantation de la membrane. L'incision est ensuite refermée. Les rats sont ensuite placés dans des cages avec nourriture et eau *ad libitum* (chaque membrane est implantée chez trois rats).

Après une semaine, les rats sont anesthésiés par un mélange Imalgène/Rumpun, et incisés afin de récupérer la membrane et le tissu épiploïque l'entourant. La membrane est alors placée dans du glutaraldéhyde à 2,5% alors que le tissu est fixé dans du paraformaldéhyde à 3% préparé dans un tampon PBS pendant 3h.

Le tissu épiploïque prélevé est inclus dans la paraffine pour analyse histologique selon le protocole suivant :

### I. Prélèvement

### I.1. Matériel

### Solutions

- 1 flacon de 500mL de Formaldéhyde à 36% (Référence SIGMA - F8775 - 500 mL).
- 1 flacon de 500 g de paraformaldéhyde en poudre (référence : SIGMA - 30525-89-4) qui doit se conserver au réfrigérateur.
- 1 flacon de 10 mL de Glutaraldéhyde à 25% (Référence: SIGMA - G5882 - 10x10 mL)
- 1 flacon de 500mL de PBS (Référence : GIBCO - 14190-094).
- 1 flacon de 500mL de PBS X10 (Référence : GIBCO - 14200-067).

### I.2. Préparation des solutions

En raison de la toxicité des produits utilisés, les étapes suivantes sont mises en oeuvre sous la hotte avec des gants.

### I.2.1. Solution de formaldéhyde à 3%

Le formaldéhyde permet de conserver le tissu entourant la membrane lors du prélèvement durant 4h maximum, il est également utilisé pour les colorations éosine/hématoxine.
- dans un flacon de 100mL, mettre 100 mL de PBS ;
- ajouter 8,3 mL de formaldéhyde à 36% ;
- mélanger.

### I.2.2. Solution de paraformaldéhyde à 8%

La solution de paraformaldéhyde à 4% peut être utilisée à la place de la solution de formaldéhyde, elle permet de fixer les tissus jusqu'à 24h et est utilisée pour la coloration immunocytochimique.
- Préparer une solution alcaline en dissolvant 1 pastille de soude dans 2 à 3 mL d'eau distillée ;
- mettre 8 g de paraformaldéhyde en suspension dans 100mL d'eau distillée ;
- sous la hotte, chauffer la suspension sur un agitateur magnétique chauffant ;
- lorsque la suspension frémit (à environ 80°C), ajouter quelques gouttes de solution alcaline jusqu'à éclaircissement total du mélange ;
- refroidir rapidement dans la glace.

Cette solution doit être utilisée immédiatement ou bien conservée à -20°C en fractions de 20 mL prêtes à l'emploi.

La solution de paraformaldéhyde à 4% est préparée par dilution avec du PBS (introduction de 20 ml de la solution de paraformaldéhyde à 8% dans 20 ml de PBS puis mélange).

### I.2.3. Solution de glutaraldéhyde à 2,5%

- Dans un flacon de 100 mL, mettre 100 mL d'eau distillée.
- Ajouter 10mL de glutaraldéhyde à 25%.

La glutaraldéhyde permet de conserver la membrane prélevée.

### II. Inclusion dans la paraffine

### II.1. matériel

- 1 flacon de 500 mL de PBS (référence : GIBCO - 14190-094).
- 1 flacon de 500 mL de PBS X10 (référence : GIBCO - 14200-067.
- 1 bouteille de 1L. de Toluène (Référence: FISHER SCIENTIFIC - code : T/2250/17), le toluène est très toxique et volatile et doit être conservé et manipulé sous la hotte.
- 1 bouteille de 1L. d'éthanol à 70%.
- 1 bouteille de 1L. d'éthanol à 95%.
- 1 bouteille de 1L. d'éthanol à 100%.
- Paraffine (Référence: TYCO/HEALTHCARE - ref: 8889501006).
- 1 cassette d'inclusion HISTOSETTE 2 standard (Référence : M.492, ref 039753 ou M.485, ref 039775 dans le catalogue Dutscher).
- 1 coupelle d'inclusion 37 x 24 mm (Référence : RICHARD-ALLAN SCIENTIFIC - catalog n° 58953)
- 3 béchers de 50mL.
- 2 béchers de 600mL.
- 1 pince.
- Aluminium.
- Gants en caoutchouc.

### II.2. Solutions

### II.2.1. PBS

- Dans un flacon vide de PBS (500mL), mettre 50mL de PBS X10 ;
- compléter avec 450mL d'eau distillée ;
- mélanger.

### II.2.2. Ethanol à 70%

- Dans une bouteille de 1L, mettre 700mL d'éthanol ;
- compléter avec 300mL d'eau distillée ;
- mélanger.

### II.2.3. Ethanol à 95%

- Dans une bouteille de 1L, mettre 950mL d'éthanol ;
- compléter avec 50mL d'eau distillée ;
- mélanger.

### II.2.4. Paraffine

- Dans les 2 béchers de 600mL, verser les pastilles de paraffine ;
- mettre les 2 béchers à 62°C-65°C dans l'étuve de la salle d'histologie ;
- conserver les béchers dans l'étuve.

### II.3. Préparation du poste de travail

Sur la paillasse : disposer 9 récipients comprenant les solutions dans l'ordre suivant : 2 récipients de PBS et 7 récipients d'éthanol.

Sous la hotte chimique, disposer 3 béchers de 50 mL de toluène et dans l'étuve, disposer 3 récipients de paraffine

### II. 4. Les bains

Les prélèvements de tissus sont traités selon les étapes suivantes :

| **ETAPES** | **SOLUTIONS** | **CONDITIONS** |
|---|---|---|
| **Fixation** | Formol 3% dans du PBS. | 2 heures mini- 4h maxi, à température ambiante. |
| **Lavages** | PBS | 2 x 10 min. |
| **Déshydratation** | Alcool à 70%. | 2 x 10 min. |
| | Alcool à 95%. | 3 x 15 min. |
| | Alcool à 100%. | 2 x 30 min. |
| **Imprégnation** | Toluène. | 3 x 15 min. |
| **Inclusion** | Paraffine. | 3 x 20 min. |

### II.5. Inclusion du tissu dans la paraffine

- Verser de la paraffine liquide dans la coupelle d'inclusion ;
- mettre le tissu à inclure dans la coupelle ;
- appliquer la cassette d'inclusion et verser par-dessus celle-ci la paraffine pour un recouvrement uniforme ;
- laisser refroidir quelques minutes à température ambiante, puis mettre au frigo pendant 24h minimum.

### III. Réalisation des coupes

### III.1. matériel

- Cassette d'inclusion.
- Microtome.
- Lames permafrost 75x25 mm (Référence : 045796, dans le catalogue Dutscher).
- 1 pipette de transfert jetable.
- 1 récipient contenant de l'eau distillée.
- 2 pinceaux : 1 brosse + 1 pinceau fin.
- 2 outils de prélèvement des coupes.
- Chauffe lame.
- 1 bouteille de 1L. d'éthanol à 95%.
- 1 bouteille de 1L. d'éthanol à 100%.
- 1 bouteille de toluène (Référence : FISHER SCIENTIFIC - code : T/2250/7)
- 5 cuves à colorations de types Hellendahl.
- Gants en caoutchouc.

### III.3. Coupes au microtome

Les coupes sont réalisées à l'aide du microtome RM2265 de Leica selon les recommandations du fabricant.

### III. 4. Déparaffinage

Cette étape permet d'éliminer la paraffine présente sur la lame tout en conservant le tissu, la lame est traitée avec les bains suivants :

| | |
|---|---|
| TOLUENE | 2 x 15 min. |
| ALCOOL 100% | 2 x 10 min. |
| ALCOOL 95% | 1 x 5 min. |
| EAU DU ROBINET | Passage rapide. |
| EAU DISTILLEE | Passage rapide. |

### IV. Coloration

### IV.1. Matériel

- Lame permafrost 76x26 mm. Avec la coupe fixée.
- 1 bouteille de 1L. d'éosine.
- 1 bouteille de 1L. d'acide alcool.
- 1 bouteille de 1L. d'eau ammoniacale.
- 1 flacon d'hæmatoxylin harris (Référence : SURGIPATH - 01562^{E}).
- 1 bouteille de 500 mL d'éthanol à 100%.
- 1 bain de Toluène.
- 1 tube de colle (Référence : EUKITT).
- Lamelle de verre 25 x 75 mm (Référence : ESCO n° 2951).
- 13 cuves à couvercles (Référence : 068506, dans le catalogue Dutscher p. 187).
- 2 paniers (Référence : 068507, dans le catalogue Dutscher p. 187).
- Papier absorbant.
- Gants en caoutchouc.
- 1 filtre à café.

### IV.2. Préparation des solutions

### IV.2.1. Solution d'éosine (1 l)

Dans une bouteille de 1L, mettre :
- Eosine 225 RA2 : 3,125g (Référence : REACTIF RAL - 312730-0100) ;
- Erythrosine RA2 : 1,875g (Référence : REACTIF RAL - 312820-0100) ;
- Eau distillée : 1L

Mélanger et filtrer à l'aide du filtre à café.

### IV.2.2. Solution d'acide alcool (1 l)

Dans une bouteille de 1L, mettre :
- Alcool à 96% : 990 mL ;
- Acide chlorhydrique (HCl) : 10 mL (Référence : PROLABO - 20.246.298.) (à manier avec des gants et des lunettes de protection)

### IV.2.3. Solution d'éthanol à 96% (1 l)

- Dans une bouteille de 1L, mettre 960 mL d'éthanol pur ;
- compléter avec 40 mL d'eau distillée et mélanger.

### IV.2.4. Solution d'eau ammoniacale à 0,20% (1 l)

Dans une bouteille de 1L, mettre :
- ammoniaque à 20% : 2,5 mL (Réf. : FISHER SCIENTIFIC - code : A/3367/17)
- Eau distillée : 1 L
puis mélanger.

### IV.3. Protocole de coloration

La coloration des tissus se fait par des bains successifs :
- trempage dans l'hæmatoxyline harris pendant 1 minute et 30 secondes ;
- deux bains dans l'eau ;
- trempage dans la solution d'acide alcool en agitant deux à trois fois ;
- deux bains dans l'eau ;
- trempage dans l'eau ammoniacale jusqu'à ce que le tissu devienne bleu ;
- un bain dans l'eau ;
- trempage dans l'éosine pendant 10 secondes ;
- un bain dans l'eau ammoniacale ;
- 3 bains successifs dans l'éthanol à 96% ;
- bain final dans le toluène.

Les bains dans l'eau et l'éthanol consistent en 2-3 agitations rapides.

Après les bains :
- appliquer la colle sur le tissu en prenant garde de ne pas le lyser ;
- appliquer la lamelle de verre et bien presser afin d'éliminer toutes les bulles d'air ;
- éliminer grossièrement le surplus de colle ;
- plonger la lame rapidement dans le toluène et le retirer aussitôt ;
- essuyer avec le papier absorbant en prenant garde de ne pas déplacer la lamelle ;
- répéter l'opération 2 à 3 fois jusqu'à élimination totale des résidus de colle.

### IV.4. Rôle des différentes solutions

L'hæmatoxyline Harris permet une coloration rouge-violet des éléments basophiles du tissu.

L'acide alcool permet une décoloration légère ainsi que l'obtention d'une teinte orangée.

L'eau ammoniacale NH4 à 0,20% permet une coloration violette des noyaux des cellules.

L'éosine permet une coloration rose-orangé des éléments acidophiles du tissu.

Les 3 bains d'éthanol à 96% permettent d'effectuer une décoloration.

Le bain de toluène permet de fixer le tissu et d'éliminer le surplus de colorant.

Le comptage des vaisseaux sanguins, la mesure de leur diamètre et la détermination de la distance entre le module et les premiers vaisseaux sanguins sont réalisés sous loupe binoculaire à l'aide d'un micromètre ; 5 champs sont comptés pour chaque coupe à raison de trois coupes par tissu.

### V. Résultats

Les clichés des coupes préparées 7 jours ou 1 mois après l'implantation des membranes sont reproduits en **Figures 4** **et** **5****.**

La **Figure 6** représente la moyenne du nombre de vaisseaux sanguins dénombrés dans 5 champs des coupes après 7 jours, 14 jours ou 1 mois pour les différentes membranes testées.

La **Figure 7** représente la distance moyenne entre la membrane semi-perméable et les premiers vaisseaux sanguins dénombrés dans 5 champs des coupes après 7 jours ou 1 mois pour les différentes membranes testées.

La **Figure 8** représente le diamètre moyen des vaisseaux sanguins mesuré dans 5 champs des coupes après 7 jours ou 1 mois pour les différentes membranes testées.

Ces essais montrent que le VEGF induit une formation de vaisseaux sanguins à long terme. L'héparine présente également un effet d'induction de la vascularisation.

Il est intéressant de noter que ces deux composés présentent un effet synergique.

## Revendications

1. Membrane semi-perméable fonctionnalisée pour la fabrication d'une chambre d'encapsulation de cellules sécrétrices d'un organe bioartificiel composée d'un support biocompatible poreux **caractérisée en ce que** :
- ledit support biocompatible poreux est préalablement traité pour que son énergie de surface soit supérieure ou égale à 50 mJ.m⁻² ;
- les pores dudit support biocompatible poreux ont une taille interne comprise entre 5 et 100 nm et
- ladite membrane semi-perméable fonctionnalisée comprend au moins deux couches comprenant chacune un polymère hydrophile et au moins une molécule biologiquement active.

2. Membrane semi-perméable fonctionnalisée selon la revendication 1, **caractérisée en ce que** le polymère hydrophile est choisi parmi les composés cellulosiques, les polyacrylamides et leurs copolymères, la polyvinylpyrrolidone (PVP) et ses copolymères, les polyvinylalcools, les copolymères de l'acétate de vinyle, les polyéthylène glycols, les polypropylène glycols, les poly(méth)acrylates hydrophiles, les polysaccharides, les polymères à base d'acide hyaluroniques et les chitosans.

3. Membrane semi-perméable fonctionnalisée selon la revendication 1 ou la revendication 2, **caractérisée en ce que** la molécule biologiquement active est choisie parmi les agents anti-inflammatoires, les agents anti-infectieux, les anesthésiques, des facteurs de croissance, les agents stimulants l'angiogenèse et/ou induisant la vascularisation, les agents inducteurs de la cicatrisation, les agents immunosuppresseurs, les agents anti-thrombotiques incluant les agents antiagrégants et les agents anticoagulants, les inhibiteurs de l'enzyme de conversion de l'angiotensine (ECA) ou les molécules stimulant l'insulino-sécrétion.

4. Membrane semi-perméable fonctionnalisée selon l'une quelconque des revendications 1 à 3, **caractérisée en ce qu'**elle comprend au moins deux couches de polymère hydrophile et de molécule biologiquement active, la première desdites couches est positionnée entre ledit support et la seconde couche et comprend un facteur de croissance cellulaire et la seconde desdites couches comprend un agent antithrombotique.

5. Membrane semi-perméable fonctionnalisée selon la revendication 4, **caractérisée en ce que** ledit facteur de croissance cellulaire est le VEGF et **en ce que** ledit agent antithrombotique est une héparine.

6. Utilisation d'une membrane selon l'une quelconque des revendications 1 à 5 pour la fabrication d'une chambre d'encapsulation de cellules sécrétrices produisant au moins une substance d'intérêt thérapeutique.

7. Chambre d'encapsulation de cellules sécrétrices produisant au moins une substance d'intérêt thérapeutique **caractérisée en ce que** ses parois sont constituées d'une membrane semi-perméable fonctionnalisée selon l'une quelconque des revendications 1 à 5 délimitant un espace susceptible de contenir les cellules sécrétrices produisant au moins une substance d'intérêt thérapeutique.

8. Organe bioartificiel **caractérisé en ce qu'**il comprend au moins une chambre d'encapsulation selon la revendication 7.

9. Organe bioartificiel selon la revendication 8, **caractérisé en ce qu'**il s'agit d'un pancréas bioartifciel contenant des cellules des îlots pancréatiques.

## Patentansprüche

1. Funktionalisierte semipermeable Membran für die Herstellung einer Einkapselungskammer für sekretierende Zellen eines bioartifiziellen Organs, die aus einem porösen biokompatiblen Träger besteht, **dadurch gekennzeichnet, dass**:
- der poröse biokompatible Träger vorher behandelt wurde, so dass seine Oberflächenenergie über oder gleich 50 mJ·m⁻² ist;
- die Poren des porösen biokompatiblen Trägers eine innere Größe zwischen 5 und 100 nm haben und
- die funktionalisierte semipermeable Membran wenigstens 2 Schichten, von denen jede ein hydrophiles Polymer umfasst, und wenigstens ein biologisch aktives Molekül umfasst.

2. Funktionalisierte semipermeable Membran nach Anspruch 1, **dadurch gekennzeichnet, dass** das hydrophile Polymer aus Celluloseverbindungen, Polyacrylamiden und ihren Copolymeren, Polyvinylpyrrolidon (PVP) und seinen Copolymeren, Polyvinylalkoholen, Vinylacetat-Copolymeren, Polyethylenglykolen, Polypropylenglykolen, hydrophilen Poly(meth)acrylaten, Polysacchariden, Polymeren auf Hyaluronsäurebasis und Chitosanen ausgewählt ist.

3. Funktionalisierte semipermeable Membran nach Anspruch 1 oder Anspruch 2, **dadurch gekennzeichnet, dass** das biologisch aktive Material aus anti-inflammatorischen Mitteln, antiinfektiösen Mitteln, Anästhetika, Wachstumsfaktoren, Mitteln, die die Angiogenese stimulieren und/oder die Vaskularisierung induzieren, Mitteln, die die Wundheilung induzieren, Immunsuppressiva, anti-thrombotischen Mitteln, einschließlich Aggregations-hemmender Mittel und Anti-Coagulantien, Inhibitoren des Angiotensin-umwandelnden Enzyms (AUE) und Molekülen, die die Insulinsekretion stimulieren, ausgewählt ist.

4. Funktionalisierte semipermeable Membran nach einem der Ansprüche 1 bis 3, **dadurch gekennzeichnet, dass** sie wenigstens zwei Schichten von hydrophilem Polymer und biologisch aktivem Molekül umfassen, wobei die erste der Schichten zwischen dem Träger und der zweiten Schicht angeordnet ist und einen Zellwachstumsfaktor umfasst und die zweite der Schichten ein antithrombotisches Mittel umfasst.

5. Funktionalisierte semipermeable Membran nach Anspruch 4, **dadurch gekennzeichnet, dass** der Zellwachstumsfaktor VEGF ist und dass das anti-thrombotische Mittel ein Heparin ist.

6. Verwendung einer Membran nach einem der Ansprüche 1 bis 5 für die Herstellung einer Einkapselungskammer für sekretierende Zellen, die wenigstens eine Substanz von therapeutischem Interesse produzieren.

7. Einkapselungskammer für sekretierende Zellen, die wenigstens eine Substanz von therapeutischem Interesse produzieren, **dadurch gekennzeichnet, dass** die Wände aus einer funktionalisierten semipermeablen Membran nach einem der Ansprüche 1 bis 5 bestehen, die einen Raum begrenzen, der geeignet ist, um die sekretierenden Zellen aufzunehmen, die wenigstens eine Substanz von therapeutischem Interesse produzieren.

8. Bioartifzielles Organ, **dadurch gekennzeichnet, dass** es wenigstens eine Einkapselungskammer nach Anspruch 7 umfasst.

9. Bioartifizielles Organ nach Anspruch 8, **dadurch gekennzeichnet, dass** es sich um ein bioartifizielles Pankreas handelt, das Pankreas-Inselzellen enthält.

## Claims

1. A functionalized semi-permeable membrane for the production of a chamber for encapsulating secretory cells of a bioartificial organ composed of a porous biocompatible support, **characterized in that**:
- said porous biocompatible support is pretreated so that its surface energy is greater than or equal to 50 mJ.m⁻²;
- the pores of said porous biocompatible support have an inner size between 5 and 100 nm, and
- said functionalized semi-permeable membrane comprises at least two layers each comprising a hydrophilic polymer and at least one biologically active molecule.

2. The functionalized semi-permeable membrane as claimed in claim 1, **characterized in that** the hydrophilic polymer is chosen from cellulose compounds, polyacrylamides and their copolymers, polyvinylpyrrolidone (PVP) and its copolymers, polyvinyl alcohols, copolymers of vinyl acetate, polyethylene glycols, polypropylene glycols, hydrophilic poly(meth)acrylates, polysaccharides, hyaluronic acid-based polymers and chitosans.

3. The functionalized semi-permeable membrane as claimed in claim 1 or claim 2, **characterized in that** the biologically active molecule is chosen from anti-inflammatory agents, anti-infective agents, anesthetics, growth factors, angiogenesis-stimulating and/or vascularization-inducing agents, wound-healing agents, immunosuppressive agents, antithrombotic agents including antiaggregation agents and anticoagulating agents, inhibitors of angiotensin-converting enzyme (ACE), or molecules stimulating insulin secretion.

4. The functionalized semi-permeable membrane as claimed in any one of claims 1 to 3, **characterized in that** it comprises at least two layers of hydrophilic polymer and biologically active molecule, the first of said layers is placed between said support and the second layer and comprises a cell growth factor and the second of said layers comprises an antithrombotic agent.

5. The functionalized semi-permeable membrane as claimed in claim 4, **characterized in that** said cell growth factor is VEGF and **in that** said antithrombotic agent is heparin.

6. The use of a membrane as claimed in any one of claims 1 to 5, for the production of a chamber for encapsulating secretory cells producing at least one substance of therapeutic interest.

7. A chamber for encapsulating secretory cells producing at least one substance of therapeutic interest, **characterized in that** its walls consist of a functionalized semi-permeable membrane as claimed in any one of claims 1 to 5 delimiting a space capable of containing the secretory cells producing at least one substance of therapeutic interest.

8. A bioartificial organ, **characterized in that** it comprises at least one encapsulation chamber as claimed in claim 7.

9. The bioartificial organ as claimed in claim 8, **characterized in that** it is a bioartificial pancreas containing cells of the pancreatic islets.
